(19) Europäisches Patentamt
European Patent Office
Office européen des brevets

(11) Publication number : **0 346 913 B1**

# (12) EUROPEAN PATENT SPECIFICATION

(45) Date of publication of patent specification : **11.11.92 Bulletin 92/46**

(51) Int. Cl.$^5$ : **C07C 37/06,** C07C 39/15, C07C 39/14

(21) Application number : **89110934.0**

(22) Date of filing : **16.06.89**

## (54) Process for the synthesis of phenyl substituted aromatic diols.

(30) Priority : **16.06.88 IT 2098388**

(43) Date of publication of application : **20.12.89 Bulletin 89/51**

(45) Publication of the grant of the patent : **11.11.92 Bulletin 92/46**

(84) Designated Contracting States : **BE CH DE ES FR GB IT LI NL SE**

(56) References cited :
**US-A- 4 088 702**
**JOURNAL OF THE AMERICAN CHEMICAL SOCIETY, vol. 75, no. 10, 21 May 1953, pages 2341-2344, American Chemical Society, Columbus,Ohio, US; R. R. BURTNER, "Synthetic Choleretics. III. Resorcinol Derivatives"**
**CHEMISCHES ZENTRALBLATT, vol. 110, no. 19, 1939, page 3883; & Journal of the American Chemical Society, vol. 61, January1939, pages 166-168; C. M. SUTER, "Die Synthese von 4- und 5-Phenylresorcinen. Das "positive" Brom des Dibromdiphenyls"**

(73) Proprietor : **HIMONT ITALIA S.r.l.**
**Foro Buonaparte, 31**
**I-20100 Milan (IT)**

(72) Inventor : **Gardano, Andrea**
**11, Via Roma, Vercelli**
**I-13039 Trino Vercellese (IT)**
Inventor : **Coassolo, Alfredo**
**34, Via Gorizia**
**I-28100 Novara (IT)**
Inventor : **Casagrande, Francesco, Dr.**
**10, Via C.M. Giulino**
**I-28100 Novara (IT)**
Inventor : **Petrini, Guido**
**11, Via Pasubio**
**I-28066 Galliate, Novara (IT)**
Inventor : **Foa', Marco, Dr.**
**19, Via del Sabbiobe**
**I-28100 Novara (IT)**
Inventor : **Chapoy, Larry Lawrence PH.D.**
**60, Via Davicini**
**I-28040 Lesa, Novara (IT)**

(74) Representative : **Beszédes, Stephan G., Dr.**
**Patentanwalt**
**Münchener Strasse 80a Postfach 1168**
**W-8060 Dachau (DE)**

EP 0 346 913 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to a process for the synthesis of phenyl substituted aromatic diols.

More particularly the present invention relates to a process for the preparation of phenyl substituted aromatic diols, starting from the corresponding cyclohexyl substituted aromatic diols, by catalytic dehydrogenation.

The aromatic diols thus obtained are important compounds for the preparation of polyesters and in particular of liquid crystalline polyesters, when the two hydroxyl groups are in parallel or coaxial position.

In particular the use of phenyl hydroquinone in the synthesis of liquid crystalline polymers is described in U.S. patents 4.159.365, 4.360.658 and 4.600.765, whereas the use of phenyl biphenol is described in Italian patent application 22746 A/87.

The phenyl substituted aromatic diols can be prepared starting from the corresponding quinones by arylation with diazonium salt of aniline (Journal of Organic Chemistry page 4071, 1977).

Such a process generally presents considerable drawbacks from a practical point of view; in fact, the starting products can not always be found on the market easily, and moreover aniline presents a potential hazard as carcinogen.

Hence the problem underlying to the invention is to create a process for the synthesis of phenyl substituted aromatic diols in which it can be started from easily accessible starting materials not injurious to health and which can be carried out in a simple way with high conversions obtaining good product yields.

Surprisingly this has been achieved by the present invention.

In fact, now it has been found that phenyl substituted aromatic diols can be obtained with high yields and conversions by a dehydrogenation process of the corresponding cyclohexyl substituted derivatives, using a supported dehydrogenation catalyst.

Hence the subject-matter of the present invention is as defined in claim 1.

In particular the dehydrogenation reaction takes place according to the following scheme:

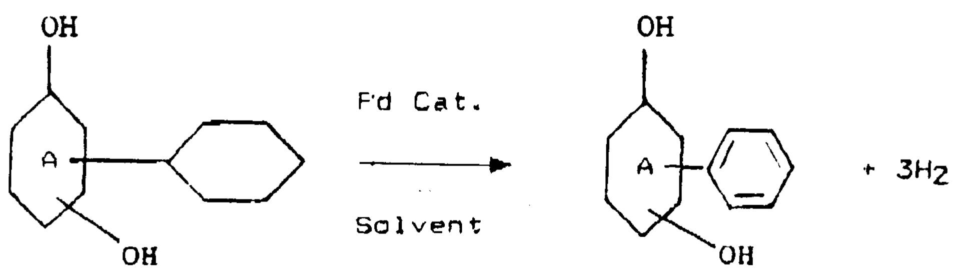


The cyclohexyl substituted aromatic diols having general formula (I) are known products, which can be obtained by cycloalkylation of aromatic diols in the presence of acid catalysts, as described in G. A. Olah "Friedel-Crafts and Related Reactions" Vol. II, Part 1, 1964.

Advantageously it is started from cyclohexyl substituted aromatic diols in which A is a phenyl, diphenyl or naphthyl radical.

Preferably the $C_1$-$C_4$ alkyl radical(s) by which the $C_6$-$C_{18}$ aromatic radical represented by A may be substituted is/are [a] methyl and/or ethyl radical(s).

It is preferred to start from products of the general formula (I), which have the two hydroxyl groups in parallel or coaxial position, particularly cyclohexylhydroquinone, 3-(cyclohexyl)-4,4'-di-(hydroxy)-diphenyl, 1-(cyclohexyl)--2,6-di-(hydroxy)-naphthalene, and 2-(cyclohexyl)-1,4-di-(hydroxy)-naphthalene.

Suitably there is used as the solvent medium such a one which, besides having the property of dissolving the reagents under the reaction conditions, is endowed with a boiling point at atmospheric pressure of at least 220°C. This latter allows to reach the temperature necessary for the dehydrogenation reaction. Temperatures ranging from 220 to 350°C are preferred.

Examples of solvents, utilizable in the process object of the present invention are: tetraethylene glycol dimethyl ether, diphenyl ether, diphenyl and polyethylene glycols having an average molecular weight ranging from 200 to 1500, such as, for instance, the ones put on the market by R.O.L., an Oil and Lubricant Refinery, under trade-names Priowax®200, Priowax®400, and Priowax®600

The concentration of the reagent having formula (I) in the solvent medium is not critical, it can range within wide limits according to the nature of the reagent, to the kind of solvent, and in general to the selected operating parameters; in particular such concentration ranges from 5 to 75% by weight, calculated on the total weight of the solution.

The reaction of dehydrogenation is carried out preferably at atmospheric pressure under a nitrogen flow to remove the developped hydrogen. Otherwise this also may be obtained by operating under vacuum.

The catalyst used in the process object of the present invention contains palladium and a suitable carrier and preferably it is used with a molar ratio reagent/Pd ranging from 50 to 10,000, particularly from 50 to 2000.

Preferred carriers are activated carbons, activated aluminas and silicas, $TiO_2$ , and MgO, particularly extruded or granulated powdered ones.

Activated carbons, particularly suitable as carriers, are known products. Such having specific surfaces ranging from 400 to 1200 $m^2/g$, particularly from 600 to 1000 $m^2/g$, are preferred.

Activated aluminas and silicas advantageously are of microspheroidal type in the extruded form or in spheres.

In the case of aluminas particularly good results were obtained when the specific surfaces were below 400 $m^2/g$, preferably between 10 and 350 $m^2/g$ and still more preferably between 30 and 300 $m^2/g$; in the case of silicas the best results were obtained when the specific surfaces ranged from 100 to 800 $m^2/g$, particularly from 200 to 500 $m^2/g$.

The pore volumes of activated aluminas preferably range from 0,2 to 1,5 $cm^3/g$ and more preferably from 0,3 to 1,3 $cm^3/g$, whereas the pore volumes of silicas preferably range from 0,5 to 2,5 $cm^3/g$ and more preferably from 1 to 2 $cm^3/g$.

In the case of granulated titanium oxide or magnesium oxide the value of the specific surface is not critical; in the former case preferably it ranges from 10 to 300 $m^2/g$, in the latter preferably from 10 to 500 $m^2/g$.

The catalyst size is not particularly binding and is bound essentially to the type of reactor to be used in the dehydrogenation reaction.

Good results can be obtained both by powdered catalysts suspended in the reaction mass and by extruded, pasted, granulated catalysts in tubular stream reactors.

The catalytic system can be prepared according to one of the general methods described in literature; for instance an acid solution of a palladium halogenide or of sodium chloropalladite ($Na_2PdCl_4$) is added to an alkaline suspension of the powdered carrier. When the addition is over, the hydrolysis compound deposited on the carrier is turned into metal by treatment with a suitable reducing agent at a temperature ranging from 20 to 100° C. Reducing agents particularly suitable to this purpose are sodium hypophosphite and sodium formate.

The solid product can be recovered by filtration and rinsed with water at temperatures ranging from 20 to 100° C, till halogenide ions are removed, and optionally dried in a stove at 100-120° C over 10-15 hours.

When a carrier of granulated, pasted or extruded type is used, a particularly advantageous method of preparation consists in letting the palladium compound be absorbed at the periphery of the carrier granules; reduction, rinsing and drying, as described hereinbefore, will follow this operation.

According to a preferred embodiment of the process object of the present invention, the catalytic system contains a small amount of alkalis so that, when it is dispersed in water, it supplies a pH over 7. Therefore it is better to treat the catalyst, before drying it, with a solution containing carbonates and/or bicarbonates of alkaline and/or alkaline-earth metals; the solution preferably has a concentration ranging from 0.1 to 5% by weight calculated on metal ion.

The palladium content of the catalytic system is not critical, but advantageously it ranges from 0.1 to 10% by weight calculated on the dried solid. Preferably the catalyst, before the reaction, is activated, at 130 -150° C for a period of time ranging from about 1 to 5 hours, with hydrogen at atmospheric pressure.

According to a practical procedure, by operating, for instance, in batch, the starting product of general formula (I) and catalyst are added to the solvent in the above mentioned ratios, in a nitrogen flow, to make the removal of developped hydrogen easier.

The reaction progress is checked by gaschromatography and the reaction is stopped preferably when the amount of cyclohexyl derivative is below 20%.

Reaction time ranges from 1 to 24 hours according to the selected operating parameters.

When the reaction is over, the catalyst can be recovered by decantation and filtration, whereas the reaction product can be recovered by known methods, for instance, by distilling the solvent or by diluting with water.

The reaction raw product can be subjected to the usual purification methods to obtain the phenyl substituted aromatic diol having the required purity, for instance, for the synthesis of polymers.

A) Preparation of the catalyst

EXAMPLE 1

60 g of microspheroidal alumina put on the market by CONDEA Company under trade-name "Puralox® SCC A-30/180 Alumina", were dispersed under stirring in a solution consisting of 4 g of sodium carbonate and

160 ml of water.
After stirring over 20 minutes, 0.3 g of palladium contained in 20 cc of a hydrochloric solution having pH 0.8, were added to the suspension, in 30 minutes. Moreover sodium ions were present in said solution, in such an amount that the atomic ratio Na/Pd was about 2.2. When the addition was over, the slurry was heated, always under stirring, up to 85° C and the temperature was kept at this value for 30 minutes; afterwards 0.4 g of sodium formate, dissolved in 10 cc of water, were added and the temperature was kept at 85° C for further 10 minutes. The solid was recovered by filtration and rinsed with water at 50-60° C till disappearance of chloride ions, afterwards it was dispersed in a solution containing 1 g of sodium carbonate in 100 ml of water and kept at rest overnight.
After filtration the obtained cake was dried at 110°C overnight. The catalyst, analysed chemically, proved to contain 0.42% by weight of palladium.

EXAMPLE 2

One operated according to example 1, while using microspheroidal commercial alumina HARSHAW® Al 3912 P. The catalyst, analysed chemically, proved to contain 0.41% by weight of palladium.

EXAMPLE 3

100 g of microspheroidal alumina Akzo® type M were calcined over 16 hours at 400° C.
A part of 60 g of calcined alumina was dispersed under stirring in a solution consisting of 8 g of sodium carbonate and 250 ml of water.
After stirring over 20 minutes 0.6 g of palladium, contained in 40 cc of a hydrochloric solution having pH 0.8, were added in 30 minutes.
Moreover sodium ions were present in said solution, in such an amount that the atomic ratio Na/Pd was about 2.2. After addition the slurry was heated, always under stirring, up to 85° C and, after 30 minutes, it was treated with 1.5 g of sodium formate dissolved in 15 ml of water and the temperature was kept at the same value for further 10 minutes.
The subsequent procedures were the same as already described in example 1.
Analysis: Pd = 0.95% by weight.

EXAMPLE 4

60 g of microspheroidal alumina, put on the market by Akzo Company as type F7, were dispersed, under stirring, in a solution consisting of 8 g of sodium carbonate in 350 ml of water.
After stirring over 20 minutes, one added 0.6 g of palladium in a solution, as described in example 3.
Then the temperature of the suspension was brought to 85° and kept at this value for 10 minutes; afterwards 1.5 g of sodium formate, dissolved in 15 ml of water, were added. After 10 minutes all the operations, described in example 1, were carried out. The dipping of the product, after rinsing, was carried out in a solution of 2.5 g of sodium carbonate and 350 ml of water.
Analysis: Pd = 0.96% by weight.

EXAMPLE 5

50 g of alumina in spheres having a diameter of 2-5 mm, put on the market by HARSHAW Company as type Filtral® SAS, were dipped in 400 ml of water for 1 hour, afterwards they were drained.
The drained carrier was introduced into a rotary baffle flask, then a hydrochloric solution having pH 2 was poured quickly onto said carrier; the hydrochloric solution contained 0.5 g of palladium and sodium ions in such an amount that the atomic ratio Na/Pd was about 2.2.
Palladium was let be absorbed slowly under a flask rotation of 10-20 rounds per minute.
After decoloration of the solution one added 1 g of sodium formate dissolved in 50 cc of water and one heated up to 70-75° C under slow rotation till the formation of gas stopped.
Then the spheres were poured into a buchner and rinsed with water at 50-60° C until disappearance of chloride ions.
At the end the spheres were dipped in a solution consisting of 4.6 g of sodium carbonate and 130 ml of water and kept at rest overnight.
After drainage the catalyst was dried at 110° C overnight.
Analysis: Pd = 0.93% by weight.

EXAMPLE 6

200 ml of distilled water were added slowly, in about 40 minutes and under stirring, to 200 ml of titanium tetraisopropylate ($Ti(OC_3H_7)_4$), put in a 1000 ml beaker. At the end the mass was stirred for 11 hours. One added 6.7 g of sodium carbonate and one stirred for further 20 minutes.
5.05 g of a solution containing about 10% by weight of palladium in the form of sodium chloropalladite and having pH 0.5, were diluted with water up to 40 ml. pH was rectified by means of 10% by weight HCl till a value of about 0.8 was reached, afterwards the palladium solution, thus obtained was added, in the course of 20 minutes, to the stirred suspension obtained by hydrolysis of titanium isopropylate.
When the addition was over one stirred for 30 minutes, then one heated to 85° C keeping the temperature at this value for 10 minutes. Then one added slowly 1.25 g of sodium formate dissolved in 15 ml of water. One stirred for further 10 minutes, afterwards one filtered and rinsed till disappearance of chloride ions.
The final product was dried at 120° C overnight.
Analysis: Pd = 1% by weight.

EXAMPLE 7

610 g of coconut coal in flakes having size of 4 x 10 mesh, put on the market by PICA Company as PICATAL 485 M, were rinsed carefully with water in order to remove the powder.
When the rinsing was over, the coal was drained carefully and then alkalized with a solution obtained by dissolving 2.45 g of sodium bicarbonate in 700 cc of distilled water.
The coal, after having been in contact with said solution for 40 minutes, was drained carefully and then poured into a rotary basket.
65.3 g of a solution of sodium chloropalladite, containing about 10% by weight of palladium and having pH 0.6, was diluted up to 700 cc with distilled water and rectified to pH 2 by means of 10% by weight HCl, afterwards 11 cc of $H_2O_2$ by 120 volumes were added there.
The palladium solution, 15 minutes after its preparation, was poured onto the coal at once, while the basket rotating slowly.
After 40 minutes a solution of 57 g of sodium hypophosphite in 100 cc of water was poured into the basket.
When the formation of hydrogen had ceased, the catalyst was taken away from the basket and rinsed by decantation till disappearance of chloride ions.
Analysis: Pd = 1% by weight.

EXAMPLE 8

50 g of magnesium oxide (light magnesium oxide MP/18 produced by General Company for Industry of Magnesia, joint-stock company) were suspended in 400 ml of water, by stirring in a 1000 ml beaker. Afterwards one added 6.7 g of sodium carbonate and subsequently slowly in the course of 20 minutes, 5.05 g of a palladium solution prepared as described in example 6. At the end of the addition one went on according to the modalities of same example 6.
Analysis: Pd = 1% by weight.

B) Carrying out the process according to the invention

EXAMPLE 9

6 g of catalyst prepared according to the modalities described in example 1, 10 g of cyclohexylhydroquinone and 30 ml of tetraethylene glycol dimethyl ether were loaded, in nitrogen atmosphere, into a 100 ml flask equipped with a mechanical stirrer, thermometer, cooler and pipe for gas inlet.
The temperature was brought to 270° C and the mixture was kept at this temperature for 5 hours under a nitrogen flow.
At the end of this period of time the gas chromatographic analysis showed a conversion of 97%. The phenylhydroquinone content in the raw product was 85% by weight. The reaction raw product, after having been cooled to room temperature, was poured into water and extracted with ethyl ether.
The ethereal solution was washed with a solution containing 10% by weight of sodium metabisulphite and with $H_2O$. After drying with $Na_2SO_4$ and evaporation of the solvent a residue was obtained weighing 9.8 g.
Such a residue was crystallized twice with toluene, thereby obtaining 7 g of practically pure phenylhydroquinone.

EXAMPLE 10

6 g of catalyst prepared according to the modalities described in example 2, 10 g of cyclohexyl hydroquinone and 30 ml of tetraethylene glycol dimethyl ether were loaded into the same apparatus and under the same conditions of example 9. The temperature was brought to 270° C and the mixture was kept at this temperature for 5 hours under a nitrogen flow. Gas chromatographic analysis of the reaction mixture showed a conversion of 91% with a phenylhydroquinone content of 82% by weight.

EXAMPLE 11

2 g of catalyst prepared according to the modalities described in example 3, 10 g of cyclohexylhydroquinone and 30 g of biphenyl were loaded into the same apparatus and under the same conditions of example 9. The temperature was brought to 250° and the mixture was kept at this temperature for 6 hours under a nitrogen flow.
Gas chromatographic analysis of the reaction mixture showed a conversion of 85% with a phenylhydroquinone content of 67% by weight.

EXAMPLE 12

2 g of catalyst prepared according to the modalities described in example 5, 10 g of cyclohexyl hydroquinone and 30 ml of tetraethylene glycol dimethyl ether were loaded into the same apparatus and under the same conditions of example 9.
The temperature was brought to 270° C and the mixture was kept at this temperature for 3 hours under a nitrogen flow. Gas chromatographic analysis of the reaction mixture showed a conversion of 85% with a phenylhydroquinone content of 68% by weight.

EXAMPLE 13

2 g of catalyst prepared according to the modalities described in example 4, 10 g of cyclohexylhydroquinone and 30 ml of tetraethylene glycol dimethyl ether were loaded into the same apparatus and under the same conditions of example 9.
The temperature was brought to 260° C and the mixture was kept at this temperature for 4 hours. Gas chromatographic analysis of the reaction mixture showed a conversion of 82%, with a phenylhydroquinone content of 61% by weight.

EXAMPLE 14

1 g of catalyst MPT5/B (5% by weight palladium on activated carbon) produced and put on the market by DUTRAL joint--stock Company, 30 g of cyclohexylhydroquinone and 40 ml of polyethylene glycol (PRIOWAX® 400 produced by ROL Company) were loaded into the same apparatus and under the same conditions of example 9.
The temperature was brought to 280° C and the mixture was kept at this temperature for 6 hours. Gas chromatographic analysis of the reaction mixture showed a conversion of 98%, with a phenylhydroquinone content of 90% by weight. By operating as described in example 6 a residue was recovered weighing 30.4 g. From this residue by crystallization from toluene (twice) 25 g of phenylhydroquinone were obtained, having a titre over 97% by weight.

EXAMPLE 15

0.5 g of MPT5/B, 10 g of cyclohexylhydroquinone and 30 ml of polyethylene glycol (PRIOWAX® 200 produced by ROL Company) were loaded into the same apparatus and under the same conditions of example 9.
The temperature was brought to 260° C and the mixture was kept at 220-260° C for 8 hours. Gas chromatographic analysis of the reaction mixture showed a conversion of 58%, with a phenylhydroquinone content of 54% by weight.

EXAMPLE 16

0.5 g of MPT5/B, 10 g of cyclohexylhydroquinone and 30 ml of polyethylene glycol (PRIOWAX®600 produced by ROL Company) were loaded into the same apparatus and under the same conditions of example 9. The temperature was brought to 290° C and the mixture was kept at this temperature for 4 hours. Gas chromatographic analysis of the reaction mixture showed a complete conversion with a phenylhydroquinone content of 85% by weight.

EXAMPLE 17

0.5 g of MPT5/B, 10 g of cyclohexylhydroquinone and 30 ml of diphenyl ether were loaded into the same apparatus and under the same conditions of example 9.
The temperature was brought to 240° C and the mixture was kept at this temperature for 6 hours. Gas chromatographic analysis of the reaction mixture showed a conversion of 90% with a phenylhydroquinone content of 70% by weight.

EXAMPLE 18

0.4 g of MPT5/B, 3.2 g of 3-cyclohexyl-4,4′-dihydroxydiphenyl and 30 ml of polyethylene glycol (PRIOWAX® 400 produced by ROL Company) were loaded into the same apparatus and under the same conditions of example 9.
The temperature was brought to 280° C and the mixture was kept at this temperature under a nitrogen flow for 5 hours. Gas chromatographic analysis of the reaction mixture showed a conversion of 98% with a content in 3-phenyl-4,4′-dihydroxydiphenyl of 93% by weight. By operating as described in example 9, 3.1 g of residue were recovered. From such a residue by crystallization from toluene 2.8 g of 3-phenyl-4,4′-dihydroxydiphenyl were obtained, having a titre over 98% by weight.

EXAMPLE 19

4 g of catalyst prepared according to the modalities described in example 6, 10 g of cyclohexylhydroquinone and 30 ml tetraethylene glycol dimethyl ether were loaded into the same apparatus and under the same conditions of example 9. The temperature was brought to 270° C and the mixture was kept at this temperature under a nitrogen flow for 5 hours. Gas chromatographic analysis of the reaction mixture showed a conversion of 59% with a phenylhydroquinone content of 43% by weight.

EXAMPLE 20

2 g of catalyst prepared according to the modalities described in example 7, 10 g of cyclohexylhydroquinone and 30 ml of tetraethylene glycol dimethyl ether were loaded into the same apparatus and under the same conditions of example 9.
The temperature was brought to 270° C and the mixture was kept at this temperature under a nitrogen flow for 6 hours. Gaschromatographic analysis of the reaction mixture showed a conversion of 97% with a phenyl hydroquinone content of 84.4% by weight.

EXAMPLE 21

3 g of catalyst prepared according to the modalities described in example 8, 10 g of cyclohexylhydroquinone and 30 ml of tetraethylene glycol dimethyl ether were loaded into the same apparatus and under the same conditions of example 9.
The temperature was brought to 260° C and the mixture was kept at this temperature under a nitrogen flow for 5 hours. Gaschromatographic analysis of the reaction mixture showed a conversion of 79% with a phenylhydroquinone content of 64.5% by weight.

## Claims

1. A process for the synthesis of phenyl substituted aromatic diols of the formula

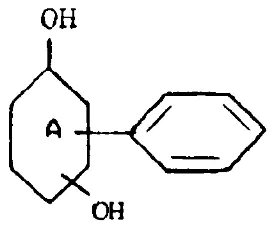

(II) ,

wherein

A represents a single, double, triple or condensed $C_6$-$C_{18}$ aromatic radical, optionally substituted by one or more $C_1$-$C_4$ alkyl radical(s),

characterized in that cyclohexyl substituted aromatic diols having the general formula

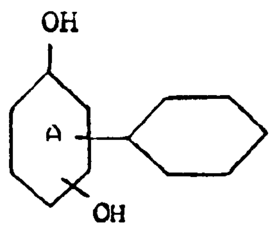

(I) ,

wherein

A is as above defined,

are dehydrogenated in the presence of a supported palladium catalyst and of a solvent medium.

2. A process according to claim 1, characterized in that there are used as cyclohexyl substituted aromatic diols having the general formula (I) such having the two hydroxyl groups in parallel or coaxial position.

3. A process according to claim 1 or 2, characterized in that there are used as cyclohexyl substituted aromatic diols having the general formula (I) cyclohexylhydroquinone, 3-(cyclohexyl)-4,4'-di-(hydroxy)-diphenyl, 1-(cyclohexyl)-2,6-di-(hydroxy)-naphthalene and 2-(cyclohexyl)-1,4-di-(hydroxy)-naphthalene.

4. A process according to any of the preceding claims, characterized in that there is used as the solvent medium such a one which, besides having the property of dissolving the reagents under the reaction conditions, is endowed with a boiling point at atmospheric pressure of at least 220°C and is selected from the group comprising tetraethylene glycol dimethyl ether, diphenyl ether, diphenyl and polyethylene glycols having an average molecular weight ranging from 200 to 1500.

5. A process according to any one of the preceding claims, characterized in that the dehydrogenation reaction is carried out at a temperature ranging from 220 to 350°C.

6. A process according to any one of the preceding claims, characterized in that a catalyst is used which comprises palladium and a carrier selected from the group comprising activated carbons, activated aluminas and silicas, $TiO_2$ and MgO.

7. A process according to any one of the preceding claims, characterized in that there is used a catalyst with a molar ration reagent/Pd ranging from 50 to 10.000.

8. A process according to claim 6, characterized in that there are used activated carbons having specific surfaces ranging from 400 to 1200 $m^2/g$.

9. A process according to claim 6, characterized in that there are used aluminas having specific surfaces below 400 $m^2/g$.

10. A process according to claim 6, characterized in that there are used silicas having specific surfaces ranging from 100 to 800 $m^2/g$.

**11.** A process according to claim 6, characterized in that there are used titanium oxides having specific surfaces ranging from 10 to 300 $m^2/g$.

**12.** A process according to any one of the preceding claims, characterized in that there are used catalysts containing an amount of alkalis obtained by treating the catalysts with a solution of carbonates and/or bicarbonates of alkaline and/or alkaline-earth metals.

**Patentansprüche**

**1.** Verfahren zu Synthese von phenylsubstituierten aromatischen Diolen der Formel

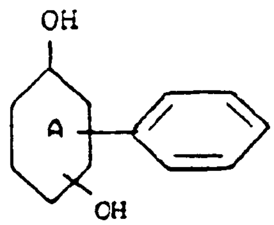

(II) ,

in der

A ein einzelnes, doppeltes, dreifaches oder kondensiertes $C_6$-$C_{18}$-aromatisches Radikal darstellt, das gegebenenfalls mit einem oder mehreren $C_1$-$C_4$-Alkylradikal(en) substituiert ist,

dadurch gekennzeichnet, daß cyclohexylsubstituierte aromatische Diole der allgemeinen Formel

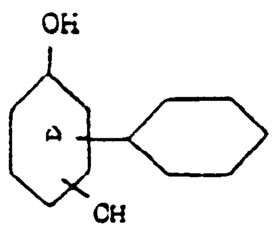

(I) ,

in der

A die oben angegebene Bedeutung hat,

in Gegenwart eines auf einem Träger befindlichen Katalysators und eines Lösungsmittelmediums dehydriert werden.

**2.** Verfahren nach Anspruch 1, dadurch gekennzeichnet, daß in den verwendeten cyclohexylsubstituierten aromatischen Diole der allgemeinen Formel (I) die zwei Hydroxylgruppen parallel oder Koaxial angeordnet sind.

**3.** Verfahren nach Anspruch 1 oder 2, dadurch gekennzeichnet, daß als cyclohexylsubstituierte aromatische Diole der allgemeinen Formel (I) das Cyclohexylhydrochinon, das 3-(Cyclohexyl)-4,4'-di-(hydroxy)-diphenyl, das 1-(Cyclohexyl)-2,6-di-(hydroxy)-naphthalin und das 2-(Cyclohexyl)-1,4-di(hydroxy)-naphthalin verwendet werden.

**4.** Verfahren nach einem der vorhergehenden Ansprüche, dadurch gekennzeichnet, daß das verwendete Lösungsmittelmedium außer der Fähigkeit zum Lösen der Reagenzien unter den Reaktionsbedingungen einen Siedepunkt von mindestens 220°C bei atmosphärischem Druck hat und aus der gruppe ausgewählt ist, zu der der Tetraethylenglykoldimethylether, Diphenylether, Diphenyl und Polyethylenglykole mit einem durchschnittlichen Molekulargewicht in Bereich von 200 bis 1500 gehören.

5. Verfahren nach einem der vorhergehenden Ansprüche, dadurch gekennzeichnet, daß die Dehydrierreaktion bei einer Temperatur im Bereich von 220 bis 350°C durchgeführt wird.

6. Verfahren nach einem der vorhergehenden Ansprüche, dadurch gekennzeichnet, daß ein Katalysator verwendet wird, der Palladium und einen Träger enthält, der aus der Gruppe ausgewählt ist, zu der Aktivkohlen, aktivierte Aluminiumoxide und Siliciumdioxide, $TiO_2$ und MgO gehören.

7. Verfahren nach einem der vorhergehenden Ansprüche, dadurch gekennzeichnet, daß ein Katalysator verwendet wird, bei dem das Molverhältnis von Reagens zu Pd im Bereich von 50 bis 10 000 liegt.

8. Verfahren nach Anspruch 6, dadurch gekennzeichnet, daß Aktivkohlen mit spezifischen Oberflächen im Bereich von 400 bis 1200 $m^2/g$ verwendet werden.

9. Verfahren nach Anspruch 6, dadurch gekennzeichnet, daß Aluminiumoxide mit spezifischen Oberflächen unter 400 $m^2/g$ verwendet werden.

10. Verfahren nach Anspruch 6, dadurch gekennzeichnet, daß Siliciumdioxide mit spezifischen Oberflächen im Bereich von 100 bis 800 $m^2/g$ verwendet werden.

11. Verfahren nach Anspruch 6, dadurch gekennzeichnet, daß Titanoxide mit spezifischen Oberflächen im Bereich von 10 bis 300 $m^2/g$ verwendet werden.

12. Verfahren nach einem der vorhergehenden Ansprüche, dadurch gekennzeichnet, daß Katalysatoren verwendet werden, die einen Gehalt an Alkalien besitzen, zu deren Bildung die Katalysatoren mit einer Lösung von Carbonaten und/oder Bicarbonaten von Alkalimetallen und/oder Erdalkalimetallen behandelt worden sind.

**Revendications**

1. Procédé pour la synthèse de diols aromatiques substitués de phényle de la formule

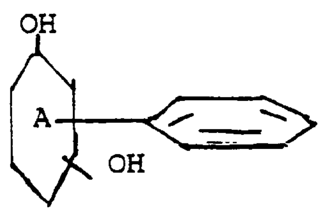

(II),

dans laquelle
A représente un radical aromatique $C_6$-$C_{18}$ simple, double, triple ou condensé, optionnellement substitué par un ou plusieurs radical/radicaux d'alcoyle $C_1$-$C_4$ caractérisé en ce que les diols aromatiques substitués de cyclohexyle ayant la formule générale

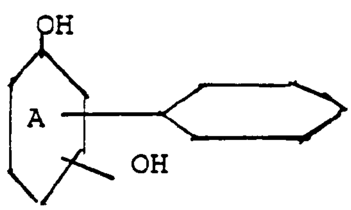

(I),

dans laquelle
A est défini comme ci-dessus,
sont déshydrogénés en présence d'un catalyseur de palladium à support et un milieu solvant.

2. Procédé selon la revendication 1, caractérisé en ce qu'on utilise comme diols aromatiques substitués de cyclohexyle de formule générale (I) ceux qui ont deux groupes d'hydroxyle en position parallèle ou co-axiale.

3. Procédé selon la revendication 1 ou 2, caractérisé en ce qu'on utilise comme diols aromatiques substitués de cyclohexyle ceux qui ont la formule générale (I) cyclohexylehydroquinone, 3-(cyclohexyle)-4,4′-di-(hydroxy)-bi-phényle, 1-(cyclohexyle)-2,6-di-(hydroxy)-naphtalène et 2-(cyclohexyle)-1,4-di-(hydroxy)-naphtalène.

4. Procédé selon l'une quelconque des revendications précédentes, caractérisé en ce qu'on utilise comme milieu solvant un qui, en plus de sa propriété de dissoudre les réactifs dans les conditions de réaction, possède un point d'ébullition à la pression atmosphérique d'au moins 220°C et qui est sélectionné à partir d'un groupe comportant du tetraéthylène glycol biméthyle-éther, de l'éther biphénilique, du biphényle et des glycols de polyéthylène ayant un poids moléculaire moyen allant de 200 à 1500.

5. Procédé selon l'une quelconque des revendications précédentes, caractérisé en ce que la réaction de déshydrogénation est exécutée à une température allant de 220°C à 350°C.

6. Procédé selon l'une quelconque des revendications précédentes, caractérisé en ce qu'un catalyseur est utilisé qui comporte du palladium et un support sélectionné à partir du groupe comportant des carbones activés, de l'alumine activée et des silices activés, du $TiO_2$ et MgO.

7. Procédé selon l'une quelconque des revendications précédentes, caractérisé en ce qu'un catalyseur est utilisé avec un ratio molaire réactif/Pd allant de 50 à 10.000.

8. Procédé selon la revendication 6, caractérisé en ce que des carbones activés sont utilisés, ayant des surfaces spécifiques allant de 400 à 1200 $m^2/g$.

9. Procédé selon la revendication 6, caractérisé en ce que des alumines sont utilisées, ayant des surfaces spécifiques inférieures à 400 $m^2/g$.

10. Procédé selon la revendication 6, caractérisé en ce que des silices sont utilisés qui ont des surfaces spécifiques allant de 100 à 800 $m^2/g$.

11. Procédé selon la revendication 6, caractérisé en ce que des oxydes de titane sont utilisés qui ont des surfaces spécifiques allant de 10 à 300 $m^2/g$.

12. Procédé selon l'une quelconque des revendications précédentes, caractérisé en ce que des catalyseurs sont utilisés, contenant une quantité d'alcalis obtenue par le traitement du catalyseur avec une solution de carbonates et/ou bi-carbonates de métaux alcalins et/ou de terre alcaline.